# EUROPÄISCHE PATENTANMELDUNG

(11) **EP 0 623 350 A1**
(43) Veröffentlichungstag der Anmeldung: **09.11.1994**
(21) Anmeldenummer: 94106197.0
(22) Anmeldetag: 21.04.1994
(51) Int. Cl.: A61K 37/02

(54) **Verwendung von Bradykinin-Antagonisten zur Herstellung eines Medikaments zur Prophylaxe oder zum Behandeln der Arteriosklerose**

(30) Priorität: 29.04.1993 DE 4314073
(71) Anmelder: HOECHST AKTIENGESELLSCHAFT, D-65929 Frankfurt am Main (DE)
(72) Erfinder: Linz, Wolfgang, Dr., D-55129 Mainz (DE); Wirth, Klaus, Dr., D-65830 Kriftel (DE); Schölkens, Bernward, Prof. Dr., D-65779 Kelkheim (DE); Breipohl, Gerhard, Dr., D-60529 Frankfurt (DE); Henke, Stephan, Dr., D-65719 Hofheim (DE); Knolle, Jochen, Dr., D-65830 Kriftel (DE)

(57) **Zusammenfassung**

Beschrieben wird die Verwendung von Bradykininantagonisten zur Prophylaxe und zum Behandeln der Arteriosklerose.

Die zu verwendenden Bradykininantagonisten zeichnen sich durch einen Gehalt an den Aminosäuren D-Tic, D-Phe, D-Dic, D-Thi oder D-Nal in Position 7 des Peptids aus.

## Beschreibung

Die Erfindung betrifft die Verwendung von Bradykinin-Atagonisten für die Behandlung und vorbeugende Behandlung der Arteriosklerose. Bradykinin und verwandte Peptide sind potente Entzündungen und Schmerzen erzeugende und vasoaktive körpereigene Substanzen.

Überraschenderweise wurde nun gefunden, daß Bradykinin-Antagonisten geeignete Therapeutika für die Prävention und Behandlung der Arteriosklerose sind.

Geeignete Bradykinin-Antagonisten sind unter anderem die Peptide der Formel I

Z - P - A - B - C - E - F - K - (D)Q - G - M - F' - I (I),

in welcher bedeuten:
- Z: a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉)-Cycloalkanoyl, oder (C₁-C₈)-Alkylsulfonyl,
   in welchen jeweils 1,2 oder 3 Wasserstoffatome gegebenenfalls durch 1,2 oder 3 gleiche oder verschiedene Reste aus der Reihe
   Carboxy, NHR(1), [(C₁-C₄)-Alkyl]NR(1) oder [(C₆-C₁₀)-Aryl-(C₁-C₄-)-alkyl]NR(1),
   wobei R(1) für Wasserstoff oder eine UrethanSchutzgruppe steht,
   (C₁-C₄)-Alkyl, (C₁-C₈)-Alkylamino, (C₆-C₁₀)-Aryl-(C₁-C₄)-alkylamino, Hydroxy, (C₁-C₄)-Alkoxy, Halogen, Di-(C₁-C₈)-alkylamino, Di-[(C₆-C₁₀)-aryl-(C₁-C₄)]-alkylamino, Carbamoyl, Phthalimido, 1,8-Naphthalimido, Sulfamoyl, (C₁-C₄)-Alkoxycarbonyl, (C₆-C₁₄)-Aryl und (C₆-C₁₄)-Aryl-(C₁-C₅)-alkyl ersetzt sind,
   oder in welchen jeweils 1 Wasserstoffatom gegebenenfalls durch einen Rest aus der Reihe
   (C₃-C₈)-Cycloalkyl, (C₁-C₆)-Alkylsulfonyl, (C₁-C₆)-Alkylsulfinyl, (C₆-C₁₄)-Aryl-(C₁-C₄)-alkylsulfonyl, (C₆-C₁₄)-Aryl-(C₁-C₄)-alkylsulfinyl, (C₆-C₁₄)-Aryl, (C₆-C₁₄)-Aryloxy, (C₃-C₁₃)-Heteroaryl und (C₃-C₁₃)-Heteroaryloxy
   und 1 oder 2 Wasserstoffatome durch 1 oder 2 gleiche oder verschiedene Reste aus der Reihe
   Carboxy, Amino, (C₁-C₈)-Alkylamino, Hydroxy, (C₁-C₄)-Alkoxy, Halogen, Di-(C₁-C₈)-alkylamino, Carbamoyl, Sulfamoyl, (C₁-C₄)-Alkoxycarbonyl, (C₆-C₁₄)-Aryl und (C₆-C₁₄)-Aryl-(C₁-C₅)-alkyl ersetzt sind;
a₂) (C₆-C₁₄)-Aryl, (C₇-C₁₅)-Aroyl, (C₆-C₁₄)-Arylsulfonyl, (C₃-C₁₃)-Heteroaryl, oder (C₃-C₁₃)-Heteroaroyl;
a₃) Carbamoyl, das gegebenenfalls am Stickstoff durch
   (C₁-C₈)-Alkyl, (C₆-C₁₄)-Aryl oder (C₆-C₁₄)-Aryl-(C₁-C₅)-alkyl substituiert sein kann;
wobei in den unter a₁), a₂) und a₃) definierten Resten die Aryl-, Heteroaryl-, Aroyl-, Arylsulfonyl- und Heteroaroyl-Gruppen gegebenenfalls durch 1,2,3 oder 4 Reste aus der Reihe
Carboxy, Amino, Nitro, (C₁-C₈)-Alkylamino, Hydroxy, (C₁-C₆)-Alkyl, (C₁-C₆)-Alkoxy, (C₆-C₁₄)-Aryl, (C₇-C₁₅)-Aroyl, Halogen, Cyano, Di-(C₁-C₈)-alkylamino, Carbamoyl, Sulfamoyl und (C₁-C₆)-Alkoxycarbonyl substituiert sind;
- P: eine direkte Bindung oder einen Rest der Formel II,

- NR(2) -(U)- CO - (II)

worin
- R(2): Wasserstoff, Methyl oder eine Urethan-Schutzgruppe bedeutet,
- U: (C₃-C₈)-Cycloalkyliden, (C₆-C₁₄)-Aryliden, (C₃-C₁₃)-Heteroaryliden, (C₆-C₁₄)-Aryl-(C₁-C₆)-alkyliden, welche gegebenenfalls substituiert sein können, oder [CHR(3)]ₙ bedeutet,
wobei n 1 - 8, vorzugsweise 1 - 6 ist,
R(3) unabhängig voneinander Wasserstoff, (C₁-C₆)-Aryl, (C₃-C₈)-Cycloalkyl, (C₆-C₁₄)-Aryl,(C₃-C₁₃)-Heteroaryl, bedeutet, die mit Ausnahme des Wasserstoffs jeweils gegebenenfalls durch
Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z, substituiertes Amidino bevorzugt für -(NH=)C-NH-Z, substituiertes Guanidino bevorzugt für -N(A')-C[=N(A')]-NH-Z und substituiertes Ureido bevorzugt für -CO-N(A')-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricylisches Ringsystem mit 2 bis 15 C-Atomen bilden;
- A: wie P definiert ist;
- B: eine basische Aminosäure in der L- oder D-Konfiguration, die in der Seitenkette substituiert sein kann;
- C: eine Verbindung der Formel III a oder III b

G'-G'-Gly (IIIa) G'-NH-(CH₂)ₚ-CO (III b)

worin
- p: 2 bis 8 ist, und
- G': unabhängig voneinander einen Rest der Formel IV

-NR(4)-CHR(5)-CO- (IV)

worin
R(4) und R(5) zusammen mit den diese tragenden Atomen ein heterocyclisches mono-, bi- oder tricyclisches Ringsystem mit 2 bis 15 C-Atomen bilden;
- E: den Rest einer neutralen, sauren oder basischen, aliphatischen oder alicyclisch-aliphatischen Aminosäure,
- F: unabhängig voneinander den Rest einer neutralen, sauren oder basischen, aliphatischen oder aromatischen Aminosäure, die in der Seitenkette substituiert sein kann, oder eine direkte Bindung;
- (D)Q: D-Tic, D-Phe, D-Dic, D-Thi oder D-Nal, die gegebenenfalls durch Halogen, Methyl oder Methoxy substituiert sein können oder einen Rest der nachstehenden Formel (V) 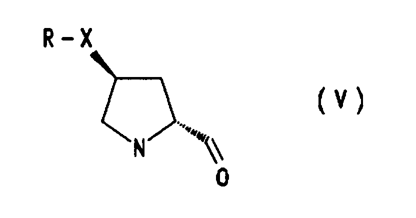 worin
- X: für Sauerstoff, Schwefel oder eine direkte Bindung steht;
- R: Wasserstoff, (C₁-C₈)-Alkyl, (C₃-C₈)-Cycloalkyl, (C₆-C₁₄)-Aryl, (C₆-C₁₄)-Aryl-(C₁-C₄)-alkyl, wobei der Alicyclus gegebenenfalls durch Halogen, Methyl oder Methoxy substituiert sein kann;
- G: wie G' oben definiert ist oder eine direkte Bindung;
- F': wie F definiert ist, einen Rest -NH-(CH₂)_{q}-, mit q = 2 bis 8, oder, falls G keine direkte Bindung bedeutet, eine direkte Bindung;
- I: -OH, -NH₂ oder NHC₂H₅;
- K: den Rest -NH-(CH₂)ₓ-CO- mit x = 1 - 4 oder eine direkte Bindung, und
- M: wie F definiert ist,
sowie deren physiologisch verträgliche Salze.

Geeignete Bradykinin-Antagonisten sind zum Beispiel beschrieben in den Patent-Veröffentlichungen EP 370 453, EP 472 220, WO 92/18155, WO 92/18156 und WO 92/17201 [Cortech; Bradykinin-Antagonisten der Formel X(BKA)ₙ, worin X ein Bindeglied, BKA die Peptidkette eines Bradykinin-Antagonisten und n eine ganze Zahl größer als 1 ist; Bradykinin-Antagonisten der Formel X(BKA); und Bradykinin-Antagonisten der Formel (Y)(X)(BKA) mit Y gleich einem Liganden, welcher ein Antagonist oder ein Agonist für einen Nicht-Bradykinin-Rezeptor ist].

Besonders geeignet sind Peptide der Formel I, in welcher bedeuten:
- Z: Wasserstoff oder wie unter a₁), a₂) oder a₃) definiert,
- P: eine Bindung oder ein Rest der Formel II

- NR(2) -(U)- CO - (II)

mit
- U: gleich CHR(3) und
R(3) wie oben defininert,
- R(2): gleich H oder CH₃,
- A: eine Bindung.

Insbesondere sind Verbindungen der Formel I bevorzugt, in der bedeuten:
- Z: Wasserstoff oder wie unter a₁), a₂) oder a₃) definiert,
- P: eine Bindung oder ein Rest der Formel II

- NR(2) -(U)- CO - (II)

mit
- U: gleich CHR(3) und
mit R(3) unabhängig voneinander Wasserstoff, (C₁-C₆)-Alkyl, (C₃-C₈)-Cycloalkyl, (C₆-C₁₄)-Aryl-(C₃-C₁₃)-Heteroaryl, die mit Ausnahme des Wasserstoffs jeweils gegebenenfalls durch
Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z und substituiertes Guanidino bevorzugt für -N(A')-C[=N(A')]-NH-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;
- R(2): gleich H oder CH₃,
- A: eine Bindung,
- (D)Q: D-Tic.

Ganz besonders geeignet ist (R)-Arginyl-(S)-arginyl-(S)-prolyl-(2S,4R)-hydroxyprolyl)glycyl-(S)-[3-(2-thienyl)alanyl]-(S)-seryl-(R)-[(1,2,3,4-tetrahydro-3-isochinolyl)carbonyl]-(2S,3aS,7aS)-[(hexahydro-2-indolinyl)carbonyl]-(S)-aginin, N-Acetat, das den INN-Namen Icatibant-Acetat trägt und auch als HOE 140 bezeichnet wird.

Die Bradykinin-Antagonisten dienen in geeigneter Verabreichungsform als Medikamente zur Behandlung der Atherosklerose.

Geeignete pharmazeutische Präparate enthalten eine wirksame Menge des Bradykinin-Antagonisten - einzeln oder in Kombination - zusammen mit einem anorganischen oder organischen pharmazeutisch verwendbaren Trägerstoff.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Hartung verbessert werden kann.

Fur die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nazallösungen kann mittels Dosierzerstäuber erfolgen oder als Nasaltropfen mit viskositätserhöhendem Anteil bzw. Nasengels oder Nasencremes.

Für die inhalative Anwendung können Vernebler oder Druckgas-Packungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Aufgrund der kurzen Halbwertszeiten einiger der beschriebenen Arzneistoffe in Körperflüssigkeiten ist der Einsatz von injizierbaren Retardzubereitungen sinnvoll. Als Arzneiformen können z. B. ölige Kristallsuspensionen, Mikrokapseln, Rods oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren oder Humanalbumin aufgebaut sein können.

Die wirksame Dosis beträgt mindestens 0.001 mg/kg/Tag, vorzugsweise mindestens 0.01 mg/kg/Tag, insbesondere mindestens 0,1 mg/kg/Tag bis höchstens 3 mg/kg/Tag, vorzugsweise bis höchstens 1 mg/kg/Tag Körpergewicht, bezogen auf einen Erwachsenen von 75 kg Gewicht.

### Untersuchung der Wirksamkeit der Bradykinin-Antagonisten:

Am Kaninchen wurde durch Fütterung einer Cholesterin-haltigen Diät arteriosklerotische Veränderungen der Gefäßwände induziert. Kaninchen wurden durch Behandlung mit HOE 140 geschützt. Arteriosklerotische Veränderungen der Gefäßwände waren stark reduziert.

### Versuchsmethode:

Der Versuch wurde mit weißen Kaninchen (New Zealand) männlichen Geschlechts mit einem Gewicht von 3-4 Kilogramm in Anlehnung an die publizierten Methoden nach A. Kisanuki, Y. Asada, K. Hatakeyama, T. Hayashi und A. Sumiyoshi: Contributions of the Endothelium to Intimal Thickening in Normocholesterolemic and Hypercholesterolemic Rabbits, Arteriosclerosis and Thrombosis (1992), 12; 1198 - 1205 und J. Cooke, A. Singer, P. Tsao, P. Zera, R.Rowan and M. Billingham: Antiatherogenic Effects of L-Arginine in the Hypercholesterolemic Rabbit(1992), 90; 1168 - 1172 durchgeführt. Nach einer Woche unter Standard-Kaninchenfutter (Altromin 2834, Lage-Lippe, Deutschland) wurden zwei Gruppen mit je 15 Kaninchen drei Monate lang mit cholesterinreicher Diät gefüttert. Die Diät enthielt 0.25% Cholesterin und 3% Kokosnußöl. Eine der beiden Gruppen erhielt zusätzlich eine subkutane Dauerinfusion von HOE 140 über implantierte osmotische Minipumpen über den gesamten Zeitraum von drei Monaten. Die Dosis betrug 100 µg/kg/Tag.

Eine weitere Gruppe von 15 Kaninchen, die nicht-atherogene normale Diät erhielt, diente als negative Kontrolle. Futter und Wasser wurden ad libitum gegeben. Der Nachweis, daß die gewählte Dosis HOE 140 die Wirkung endogener Kinine hemmen kann, wurde über die Hemmung der blutdrucksenkenden Wirkung von exogenem Bradykinin geführt. Am Ende des Versuches, nach dreimonatiger Behandlung, wurden die Tiere narkotisiert und der basale Blutdruck gemessen. Der basale Blutdruck lag zwischen 100 und 110 mm Hg. Nach der Messung des basalen Blutdrucks wurde Bradykinin intraarteriell als Bolus in einer Dosis von 10 ng injiziert. Bei den Tieren mit normalem Futter führte dies zu einem Blutdruckabfall von 21 mm Hg, bei den Tieren mit atherogener Diät betrug der Blutdruckabfall 24 mm Hg. Bei Kaninchen mit atherogener Diät, die mit HOE 140 behandelt wurden, fiel der Blutdruck nur noch um 3.7 mm Hg, was eine effektive Hemmung der Bradykininwirkung durch HOE 140 anzeigte.

Danach wurden die Kaninchen mit einer hohen Dosis von Pentobarbital (40 mg/kg) getötet und eine komplette Autopsie durchgeführt. Die atherosklerotischen Plaques wurden durch Öl-Rot O (C₂₆H₂₄N₄O) sichtbar gemacht, ihr Ausmaß mittels eines Bildanalysators quantifiziert und die prozentuale Plaquegröße in der Aorta bestimmt. Weiterhin wurden die Cholesterinfraktionen (HDL, LDL und VLDL) im Serum mit Hilfe der Ultrazentrifugation bestimmt.

### Ergebnisse

### Auswertung der arteriosklerotischen Veränderungen:

Aorten von Kaninchen unter normalem Futter wiesen keinerlei arteriosklerotische Gefäßveränderungen auf. Aorten von Kaninchen mit cholesterinhaltigen Futter zeigten ausprägte Gefäßveränderungen in Form von atherosklerotischen Plaques. Überraschenderweise zeigte HOE 140 antiatherosklerotische Wirkungen: Atherosklerotische Veränderungen des Aortenbogens und der Aortenabschnitte in Brust und Bauch waren bei weitem geringer bei den Tieren, die zur atherogenen Diät HOE 140 erhielten (Tabelle 1).

Wirkung auf Serumlipide: LDL und VLDL waren durch HOE 140 stark erniedrigt und von Lipidwerten der Kontrollen mit normalem, nicht-atherogenem Futter kaum zu unterscheiden (Tabelle 2). HDL-Werte waren bei den Tieren mit cholesterinreichem Futter deutlich erhöht. Die Behandlung mit HOE 140 zeigte keinen Einfluß auf diesen Parameter.

### Schlußfolgerung:

Bradykinin-Antagonisten senken Serumlipide und besitzen ausgeprägte antiatherosklerotische Wirkungen. Aufgrund dieser Wirkungen sind sie für die Therapie und vorbeugende Behandlung der Arteriosklerose geeignet.

**Tabelle 1**

| Plaques in der Aorta: Fläche in Prozent der Intimafläche (Mittelwerte ± S.E.M.) | | | |
|---|---|---|---|
| | normale Diät | atherogene Diät | atherogene Diät plus HOE 140 |
| Aortenbogen | 0 | 44.3 ± 4.92 | 23.4 ± 5.4 |
| Thorakale Aorta | 0 | 12.3 ± 4.0 | 1.6 ± 1.1 |
| Abdominelle Aorta 1* | 0 | 10.8 ± 3.2 | 1.4 ± 0.6 |
| Abdominelle Aorta 2* | 0 | 14.6 ± 1.9 | 7.0 ± 1.5 |

| | | | |
|---|---|---|---|
| * 1 bzw. 2 = erster bzw. zweiter Abschnitt | | | |

**Tabelle 2**

| Serum Lipoprotein Fraktionen (µg/ml) (Mittelwerte ± S.E.M.) | | | |
|---|---|---|---|
| | normale Diät | atherogene Diät | atherogene Diät plus HOE 140 |
| LDL | 257 ± 63 | 2950 ± 329 | 168 ± 21 |
| VLDL | 330 ± 64 | 2837 ± 163 | 250 ± 12 |
| HDL | 2871 ± 23 | 5703 ± 610 | 4981 ± 576 |

## Patentansprüche

1. Verwendung eines Bradykinin-Antagonisten oder eines seiner physiologisch verträglichen Salze zur Herstellung eines Medikaments zur Prophylaxe oder zum Behandeln der Arteriosklerose.

2. Verwendung nach Anspruch 1 eines Bradykinin-Antagonisten der Formel I
Z - P - A - B - C - E - F - K - (D)Q - G - M - F' - I (I),
in welcher bedeuten:
Z
a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉)-Cycloalkanoyl, oder (C₁-C₈)-Alkylsulfonyl,
in welchen jeweils 1,2 oder 3 Wasserstoffatome gegebenenfalls durch 1,2 oder 3 gleiche oder verschiedene Reste aus der Reihe
Carboxy, NHR(1), [(C₁-C₄)-Alkyl]NR(1) oder [(C₆-C₁₀)-Aryl-(C₁-C₄)-alkyl]NR(1),
wobei R(1) für Wasserstoff oder eine Urethan-Schutzgruppe steht,
(C₁-C₄)-Alkyl, (C₁-C₈)-Alkylamino, (C₆-C₁₀)-Aryl-(C₁-C₄)-alkylamino, Hydroxy, (C₁-C₄)-Alkoxy, Halogen, Di-(C₁-C₈)-alkylamino, Di-[(C₆-C₁₀)-aryl-(C₁-C₄)]-alkylamino, Carbamoyl, Phthalimido, 1,8-Naphthalimido, Sulfamoyl, (C₁-C₄)-Alkoxycarbonyl, (C₆-C₁₄)-Aryl und (C₆-C₁₄)-Aryl-(C₁-C₅)-alkyl ersetzt sind,
oder in welchen jeweils 1 Wasserstoffatom gegebenenfalls durch einen Rest aus der Reihe
(C₃-C₈)-Cycloalkyl, (C₁-C₆)-Alkylsulfonyl, (C₁-C₆)-Alkylsulfinyl, (C₆-C₁₄)-Aryl-(C₁-C₄)-alkylsulfonyl, (C₆-C₁₄)-Aryl-(C₁-C₄)-alkylsulfinyl, (C₆-C₁₄)-Aryl, (C₆-C₁₄)-Aryloxy, (C₃-C₁₃)-Heteroaryl und (C₃-C₁₃)-Heteroaryloxy
und 1 oder 2 Wasserstoffatome durch 1 oder 2 gleiche oder verschiedene Reste aus der Reihe
Carboxy, Amino, (C₁-C₈)-Alkylamino, Hydroxy, (C₁-C₄)-Alkoxy, Halogen, Di-(C₁-C₈)-alkylamino, Carbamoyl, Sulfamoyl, (C₁-C₄)-Alkoxycarbonyl, (C₆-C₁₄)-Aryl und (C₆-C₁₄)-Aryl-(C₁-C₅)-alkyl ersetzt sind;
a₂) (C₆-C₁₄)-Aryl, (C₇-C₁₅)-Aroyl, (C₆-C₁₄)-Arylsulfonyl, (C₃-C₁₃)-Heteroaryl, oder (C₃-C₁₃)-Heteroaroyl;
a₃) Carbamoyl, das gegebenenfalls am Stickstoff durch
(C₁-C₈)-Alkyl, (C₆-C₁₄)-Aryl oder (C₆-C₁₄)-Aryl-(C₁-C₅)-alkyl substituiert sein kann;
wobei in den unter a₁), a₂) und a₃) definierten Resten die Aryl-, Heteroaryl-, Aroyl-, Arylsulfonyl- und Heteroaroyl-Gruppen gegebenenfalls durch 1,2,3 oder 4 Reste aus der Reihe
Carboxy, Amino, Nitro, (C₁-C₈)-Alkylamino, Hydroxy, (C₁-C₆)-Aryl, (C₁-C₆)-Alkoxy, (C₆-C₁₄)-Aryl, (C₇-C₁₅)-Aroyl, Halogen, Cyano, Di-(C₁-C₈)-alkylamino, Carbamoyl, Sulfamoyl und (C₁-C₆)-Alkoxycarbonyl substituiert ist;
P für eine direkte Bindung steht oder einen Rest der Formel II bedeutet,
- NR(2) -(U)- CO - (II)
worin
R(2) Wasserstoff, Methyl oder eine Urethan-Schutzgruppe bedeutet,
U (C₃-C₈)-Cycloalkyliden, (C₆-C₁₄)-Aryliden, (C₃-C₁₃)-Heteroaryliden, (C₆-C₁₄)-Aryl-(C₁-C₆)-alkyliden, welche gegebenenfalls substituiert sein können, oder [CHR(3)]ₙ bedeutet,
wobei n 1 - 8, vorzugsweise 1 - 6 ist,
R(3) unabhängig voneinander Wasserstoff, (C₁-C₆)-Aryl, (C₃-C₈)-Cyloalkyl, (C₆-C₁₄)-Aryl, (C₃-C₁₃)-Heteroaryl, bedeutet, die mit Ausnahme des Waserstoffs jeweils gegebenenfalls durch
Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pridyl, 3-Pyridyl oder Cyclohexyl monosubstituiert sind,
wobei substituiertes Amino bevorzugt für -N(A')-Z, substituiertes Amidino bevorzugt für -(NH=)C-NH-Z, substituiertes Guanidino bevorzugt für -N(A')-C[=N(A')]-NH-Z und substituiertes Ureido bevorzugt für -CO-N(A')-Z stehen, in denen A' unabhbägig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;
A wie P definiert ist;
B eine basische Aminosäure in der L- oder D-Konfiguration, die in der Seitenkette substituiert sein kann;
C eine Verbindung der Formel III a oder III b
G'-G'-Gly (III a) G'-NH-(CH₂)ₚ-CO (III b)
worin
p 2 bis 8 ist, und
G, unabhängig voneinander einen Rest der Formel IV
-NR(4)-CHR(5)-CO- (IV)
worin
R(4) und R(5) zusammen mit den diese tragenden Atomen ein heterocyclisches mono-, bi- oder tricyclisches Ringsystem mit 2 bis 15 C-Atomen bilden;
E den Rest einer neutralen, sauren oder basischen, aliphatischen oder alicyclisch-aliphatischen Aminosäure;
F unabhängig voneinander den Rest einer neutralen, sauren oder basischen, aliphatischen oder aromatischen Aminosäure, die in der Seitenkette substituiert sein kann, oder für eine direkte Bindung;
(D)Q D-Tic, D-Phe, D-Dic, D-Thi oder D-Nal, die gegebenenfalls durch Halogen, Methyl oder Methoxy substituiert sein können oder einen Rest der nachstehenden Formel (V) 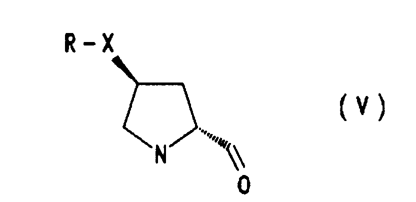 worin
X für Sauerstoff, Schwefel oder eine direkte Bindung steht;
R Wasserstoff, (C₁-C₈)-Alkyl, (C₃-C₈)-Cycloalkyl, (C₆-C₁₄)-Aryl, (C₆-C₁₄)-Aryl-(C₁-C₄)-alkyl, wobei der Alicyclus gegebenenfalls durch Halogen, Methyl oder Methoxy substituiert sein kann;
G wie G' oben definiert ist oder eine direkte Bindung;
F' wie F definiert ist, oder einen Rest -NH-(CH₂)_{q}-, mit q = 2 bis 8, oder, falls G keine direkte Bindung bedeutet, eine direkte Bindung;
I -OH, -NH₂ oder NHC₂H₅;
K den Rest -NH-(CH₂)ₓ-CO- mit x = 1 - 4, oder eine direkte Bindung, und
M wie F definiert ist,
oder eines seiner physiologisch verträglichen Salze.

3. Verwendung nach Anspruch 2 eines Bradykinin-Antagonisten der Formel I, in welcher bedeuten:
Z Wasserstoff oder wie unter a₁), a₂) oder a₃) definiert,
P eine Bindung oder einen Rest der Formel II
- NR(2) -(U)- CO - (II)
mit
U gleich CHR(3) und
R(3) wie oben defininert,
R(2) gleich H oder CH₃,
A eine Bindung.

4. Verwendung nach Anspruch 2 eines Bradykinin-Antagonisten der Formel I, in der bedeuten:
Z Wasserstoff oder wie unter a₁), a₂) oder a₃) definiert,
P eine Bindung oder ein Rest der Formel II
- NR(2) -(U)- CO - (II)
mit
U gleich CHR(3) und
mit R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, die mit Ausnahme des Wasserstoffs jeweils gegebenenfalls durch
Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z und substituiertes Guanidino bevorzugt für -N(A')-C[=N(A')]-NH-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;
R(2) gleich H oder CH₃,
A eine Bindung,
(D)Q D-Tic.

5. Verwendung eines Bradykinin-Antagonisten zur Herstellung eines Medikaments zur Prophylaxe oder zum Behandeln der Arteriosklerose nach Anspruch 2, dadurch gekennzeichnet, daß der Bradykinin-Antagonist (R)-Arginyl-(S)-arginyl-(S)-prolyl-(2S,4R)-hydroxyprolyl)glycyl-(S)-[3-(2-thienyl)alanyl]-(S)-seryl-(R)-[(1,2,3,4-tetrahydro-3-isochinolyl)carbonyl]-(2S,3aS,7aS)-[(hexahydro-2-indolinyl)carbonyl]-(S)-arginin, N-Acetat ist.
